Europäisches Patentamt

(19) European Patent Office

Office européen des brevets

(11) Publication number: **0 027 391**
**B1**

(12) **EUROPEAN PATENT SPECIFICATION**

(45) Date of publication of patent specification: 09.02.83

(51) Int. Cl.³: **C 07 C 51/48,**
C 07 C 57/04,
C 07 C 45/80,
C 07 C 47/22,
C 07 C 121/32

(21) Application number: **80303657.3**

(22) Date of filing: **16.10.80**

(54) Dehydration of water soluble monomers.

(43) Date of publication of application:
22.04.81 Bulletin 81/16

(45) Publication of the grant of the patent:
09.02.83 Bulletin 83/6

(84) Designated Contracting States:
BE CH DE FR GB IT LI LU NL SE

(56) References cited:
DE - A - 1 102 719

CHEMICAL ABSTRACTS, vol. 84 (1976) no. 20,
17th May 1976, abstract no 137596c, page 121
Columbus, Ohio, US

(73) Proprietor: THE STANDARD OIL COMPANY
Midland Building
Cleveland, Ohio 44115 (US)

(72) Inventor: Hardman, Harley Foch
4989 Delevan Drive
Lyndhurst Ohio 44124 (US)
Inventor: Schwerko, Albert Peter
36695 Timberland Drive
Solon Ohio 44139 (US)

(74) Representative: Smith, Sydney et al,
Elkington and Fife High Holborn House 52/54
High Holborn
London WC1V 6SH (GB)

Courier Press, Leamington Spa, England

## Dehydration of water soluble monomers

This invention relates to the dehydration of water-soluble monomers by the utilization of carbon dioxide.

It is well known in the art that carbon dioxide can be used for extraction of various petroleum fractions. Liquid carbon dioxide is advantageous because of its low cost, non-corrosiveness, non-toxicity and ease of recovery from the extract or raffinate phases. U.S. Patent No. 2,029,120 discloses the separation of unsaturated hydrocarbons from gas mixtures using liquid carbon dioxide.

U.S. 2,631,966 shows the separation of various lubricating oils with carbon dioxide. As noted therein, liquid $CO_2$ has certain unusual miscibility relations with hydrocarbons. For example, it dissolves aliphatic and monocyclic aromatic hydrocarbons in preference to polycyclics of the same boiling range.

Typically, $CO_2$ has not been used alone, but as an aid to other solvents for extraction purposes. These solvents include liquid sulphur dioxide (U.S. 2,034,495) acetone (2,246,227) and furfural or phenol (2,281,865). A broad description of various solvents that can be used with carbon dioxide can be found in U.S. 2,631,966.

Liquid $CO_2$ has also been utilized to remove the solvents themselves from the extract phase, as can be found in U.S. 2,646,387.

The ability of $CO_2$ to function in the above systems will depend upon the solubility of $CO_2$ with other substances. "Ternary Systems of Liquid Carbon Dioxide" by Alfred W. Frances, *Journal of Physical Chemistry* 58, 1099 (1954) discloses the solubilities of 261 substances with carbon dioxide.

None of the processes above, however, discloses the ability or process for the dehydration of an aqueous solution by using $CO_2$.

There are a large number of processes for the production of certain organic chemicals that produce water as a by-product or use steam as a diluent during the reaction. Among these processes is the production of acrylic acid. The aqueous solution resulting from the reaction normally contains from 30—60% acrylic acid with the remainder being mostly water. Various methods have been proposed for removing this water, such as the addition of certain drying agents like calcium chloride as found in U.S. 2,922,815.

Other processes, such as for the production of acrylonitrile, use water as an aid in distilling acrylonitrile from the reactor effluent. This produces an aqueous solution of acrylonitrile that must be dehydrated.

The present invention provides a process for separating water from such aqueous solutions of organic chemicals that can achieve purities upwards of 99%.

The invention provides a process for the separation of water from an aqueous solution containing an organic chemical having a distribution coefficient between carbon dioxide and water greater than 1 miscible with $CO_2$ and comprising the steps of:

a) contacting the aqueous solution with liquid carbon dioxide at a temperature below 31.1°C and at a pressure between 25.53 to 73.22 bar gauge to form an extract phase of the organic chemical, carbon dioxide and some water, and a raffinate phase containing water;

b) separating the extract phase from the raffinate phase;

c) crystallizing by cooling and separating carbon dioxide hydrate from the extract phase; and

d) evaporating the carbon dioxide.

It is possible to reverse steps c) and d) above such that the carbon dioxide solvent is partly evaporated prior to the crystallization and separation of the carbon dioxide hydrate.

The above process is especially suited for recovery of heat sensitive materials, such as acrylic acid, methacrylic acid, acrolien, methacrolien acrylonitrile or methacrylonitrile. However, it may be applied to any material having a favourable distribution coefficient between carbon dioxide and water. This distribution coefficient is the ratio of concentration of organics in carbon dioxide and water. If this ratio is greater than 1, then the coefficient can be considered favourable.

A major advantage of the present invention is that operations are conducted at temperatures below 31.1°C limiting thermal degradation or polymerization of the heat sensitive materials. This temperature limitation is because the critical temperature of carbon dioxide is 31.1°C.

The temperatures employed can vary depending on the step in the process. For example, one would not want to contact an aqueous solution containing acrylic acid at a temperature much below 10°C. Temperatures below this may cause crystals to form. However, during the crystallizing step, temperatures of about 0°C can be used. The exact temperature will depend on factors such as the pressure used and the specific organic chemical to be dehydrated.

Since carbon dioxide is being utilized in a liquid state, pressures in the range of 25.53 to 73.22 bar gauge (370 to 1060 psig) are required. The exact pressure will depend on the temperature, the specific organic chemical, the amount of organics being separated, and the $CO_2$/aqueous solution weight ratio. A preferred range is 41.40 bar gauge to 72.46 bar gauge (600 to 1050 psig). As an example, in the separation of acrylic acid from water, pressures of from 31.05 bar gauge of 58.66 bar gauge (450 to 850 psig) are normally used.

The liquid $CO_2$ can be contacted with the aqueous solution either by batch operation or by continuous extraction. It is preferred to use continuous countercurrent extraction to perform the contacting and separation of the extract and raffinate phases. Such extraction processes are well known in the art.

Carbon dioxide hydrate is crystallized by cooling the extract phase to a temperature where the white crystals are formed and precipitate. For acrylic acid systems, this temperature is approximately 0°C.

After the crystals have been separated, which can be accomplished in a normal manner such as by filtration, the remaining carbon dioxide solvent is removed by simple reduction in pressure. This flashes off or evaporates the $CO_2$.

It is also possible to first evaporate part of the $CO_2$ and then cool the remaining solution to crystallize and separate the carbon dioxide hydrate. There should be about 0.31 g $CO_2$/gm of water remaining to form the hydrate. Some additional $CO_2$ should be present to prevent the product from crystallizing during the extraction.

The weight ratio of $CO_2$ to the aqueous solution will vary, dependent upon the amount of organics to be separated. Typically, this ratio is 2:1 but can be as low as 0.5:1 and as high as 10 or 20:1. A ratio between 1:1 and 3:1 is preferred.

The carbon dioxide can be recovered, liquified and reused in the process.

The following Examples illustrate the invention:—

### Example 1

A 31.2 wt. percent solution of acrylic acid in water was contacted with liquid $CO_2$ at 25°C and 57.97 bar gauge (840 psig). The weight ratio of $CO_2$:acrylic acid solution was 1.97. After equilibration, the lower aqueous phase was withdrawn, and the $CO_2$ phase was cooled to 0°C, when white crystals were formed. The crystals were separated from the liquid, and $CO_2$ evaporated from the liquid by gradual reduction of pressure. The remaining non-volatile liquid was found to be acrylic acid containing 0.9% water and a trace of dissolved $CO_2$.

### Example 2

A 34 wt. percent solution of acrylic acid in water was extracted with liquid $CO_2$ in a countercurrent extraction unit having six theoretical stages of contacting. Conditions were 25°C, 58.66 bar gauge (850 psig), solvent/feed (Wt.) = 3. Analysis of the extract and raffinate showed that 95.3% of the acrylic acid fed was recovered in the extract. The extract composition — after $CO_2$ removal — was 92.5% acrylic acid, 7.5% water. Cooling the extract to 0°C and removing the crystals so formed yielded a product of composition 99% acrylic acid, 1% water (solvent free basis).

As can be seen above, extremely high purities of the organic chemicals can be achieved through the use of the present invention.

## Claims

1. A process for the separation of water from an aqueous solution containing an organic chemical having a distribution coefficient between carbon dioxide and water greater than 1 miscible with $CO_2$ and comprising the steps of:
   a) contacting the aqueous solution with liquid carbon dioxide at a temperature below 31.1°C and at a pressure between 25.53 to 73.22 bar gauge to form an extract phase of the organic chemical, carbon dioxide and some water, and a raffinate phase containing water;
   b) separating the extract phase from the raffinate phase;
   c) crystallizing by cooling and separating carbon dioxide hydrate from the extract phase; and
   d) evaporating the carbon dioxide.

2. A process as claimed in claim 1 characterised in that the contacting of step a) occurs at a pressure between 41.40 bar to 72.46 bar gauge (600 and 1050 psig).

3. A process as claimed in claim 1 or claim 2 characterised in that the carbon dioxide hydrate of step c) is separated by filtration.

4. A process as claimed in any of claims 1 to 3 characterised in that $CO_2$ solvent is evaporated by reduction in pressure.

5. A process as claimed in any of claims 1 to 4 characterised in that the organic chemical is one of the following, namely acrylic acid, methacrylic acid, acrolein, methacrolein, acrylonitrile or methacrylonitrile.

6. A process as claimed in any of claims 1 to 5 characterised in that the ratio of carbon dioxide to the aqueous solution is 1:1 to 3:1.

7. A process as claimed in any of claims 1 to 6 characterised in that the contacting of step a) and the separation of step b) occurs in an extraction column.

8. A process as claimed in any of claims 1 to 7 characterised in that stages (c) and (d) are reversed in time and in which a part of the carbon dioxide is evaporated from the extract phase prior to crystallization and separation of the carbon dioxide hydrate from the extract phase.

9. A process for the separation of water from an aqueous solution containing an organic chemical miscible with $CO_2$ and having a distribution coefficient between carbon dioxide and water greater than 1 comprising the steps of:
   a) contacting the aqueous solution with liquid carbon dioxide at a temperature below 31.1°C and at a pressure between 25.53 to 73.22 bar gauge to form an extract phase of the organic chemical, carbon dioxide and some water, and a raffinate phase containing water;
   b) separating the extract phase from the raffinate phase;
   c) evaporating a part of the carbon dioxide from the extract phase; and

d) crystallizing and separating carbon dioxide hydrate from the extract phase.

## Patentansprüche

1. Verfahren zur Abtrennung von Wasser von einer wäßrigen Lösung, die eine organische Chemikalie enthält, die einen Verteilungskoeffizienten zwischen Kohlenoxid und Wasser von größer als 1 aufweist, mit $CO_2$ mischbar ist, das folgende Stufen umfaßt:
a) Kontakt der wäßrigen Lösung mit flüssigem Kohlenoxid bei einer Temperatur unter 31,1°C und einem Überdruck von 25,53 bis 73,22 bar, unter Bildung einer Extraktphase der organischen Chemikalie von Kohlendioxid und etwas Wasser und einer Raffinatphase, die Wasser enthält;
b) Abtrennen der Extraktphase von der Raffinatphase;
c) Kristallisieren und Abtrennen von Kohlendioxid-Hydrat von der Extraktphase durch Kühlen; und
d) Verdampfen des Kohlendioxids.

2. Verfahren nach Anspruch 1, dadurch gekennzeichnet, daß der Kontakt der Stufe (a) bie einem Druck von 41,40 bar bis 72,46 bar Überdruck (600 bis 1050 psig) erfolgt.

3. Verfahren nach Anspruch 1 oder Anspruch 2, dadurch gekennzeichnet, daß das Kohlendioxid-Hydrat der Stufe (c) durch Filtrieren abgetrennt wird.

4. Verfahren nach einem der Ansprüche 1 bis 3, dadurch gekennzeichnet, daß das $CO_2$-Lösungsmittel durch Verringern des Drucks verdampft wird.

5. Verfahren nach einem der Ansprüche 1 bis 4, dadurch gekennzeichnet, daß die organische Chemikalie eine der folgenden ist, nämlich Acrylsäure, Methacrylsäure, Acrolein, Methacrolein, Acrylnitril oder Methacrylnitril.

6. Verfahren nach einem der Ansprüche 1 bis 5, dadurch gekennzeichnet, daß das Verhältnis von Kohlendioxid zu der wäßrigen Lösung 1 : 1 bis 3 : 1 beträgt.

7. Verfahren nach einem der Ansprüche 1 bis 6, dadurch gekennzeichnet, daß der Kontakt der Stufe (a) und die Trennung der Stufe (b) in einer Extraktionskolonne erfolgen.

8. Verfahren nach einem der Ansprüche 1 bis 7, dadurch gekennzeichnet, daß die Stufen (c) und (d) zeitlich ausgetauscht werden und ein Teil des Kohlendioxids aus der Extraktphase vor dem Kristallisieren und der Abtrennung des Kohlendioxid-Hydrats von der Extraktphase verdampft wird.

9. Verfahren zur Abtrennung von Wasser von einer wäßrigen Lösung, die eine organische Chemikalie enthält, die mit $CO_2$ mischbar ist und einen Verteilungskoeffizienten zwischen Kohlendioxid und Wasser von größer als 1 aufweist, das folgende Stufen umfaßt:
a) Kontakt der wäßrigen Lösung mit flüssigem Kohlendioxid bei einer Temperatur unter 31,1°C und bei einem Überdruck von 25,53 bis 73,22 bar unter Bildung einer Extraktphase der organischen Chemikalie, von Kohlendioxid und etwas Wasser, und einer Raffinatphase, die Wasser enthält;
b) Abtrennen der Extraktphase von der Raffinatphase;
c) Verdampfen eines Teils des Kohlendioxids von der Extraktphase; und
d) Kristallisieren und Abtrennen von Kohlendioxid-Hydrat aus der Extraktphase.

## Revendications

1. Procédé pour la séparation de l'eau à partir d'une solution aqueuse contenant un produit chimique organique ayant un coefficient de distribution entre le dioxyde de carbone et l'eau supérieur à 1, miscible avec le $CO_2$, et comprenant les étapes consistant à:
a) mettre en contact la solution aqueuse avec le dioxyde de carbone liquide à une température inférieure à 31,1°C et à une pression relative comprise entre 25,53 bars et 73,22 bars pour former une phase d'extraction renfermant le produit chimique organique du dioxyde de carbone et un peu d'eau, et une phase résiduelle contenant l'eau;
b) séparer la phase d'extraction de la phase résiduelle;
c) cristalliser par refroidissement et séparer l'hydrate de dioxyde de carbone à partir de la phase d'extraction; et
d) évaporer le dioxyde de carbone.

2. Procédé selon la revendication 1, caractérisé par le fait que la mise en contact de l'étape a) s'effectue à une pression relative comprise entre 41,40 bars et 72,46 bars (600 à 1050 psig).

3. Procédé selon l'une des revendications 1 ou 2, caractérisé par le fait que l'hydrate de dioxyde de carbone dans l'étape c) est séparé par filtration.

4. Procédé selon l'une des revendications 1 à 3, caractérisé par le fait que le solvent $CO_2$ est évaporé par réduction de pression.

5. Procédé selon l'une des revendications 1 à 4, caractérisé par le fait que le produit chimique organique est l'un des produits suivants, à savoir, l'acide acrylique, l'acide métacrylique, l'acroléine, la métacroléine ou le métacrylonitrile.

6. Procédé selon l'une des revendications 1 à 5 caractérisé par le fait que le rapport du dioxyde de carbone à la solution aqueuse est de 1:1 à 3:1.

7. Procédé selon l'une des revendications 1 à 6, caractérisé par le fait que la mise en contact de l'étape a) et la séparation de l'étape b) s'effectuent dans une colonne d'extraction.

8. Procédé selon l'une des revendications 1 à 7, caractérisé par le fait que les étapes c) et d) sont interverties dans le temps et dans lequel une partie du dioxyde de carbone est évaporé à partir de la phase d'extraction avant la cristallisation et la séparation de l'hydrate de dioxyde de carbone à partir de la phase d'extraction.

9. Procédé pour la séparation de l'eau à partir d'une solution aqueuse contenant un produit chimique organique miscible avec le $CO_2$, et ayant un coefficient de distribution entre le dioxyde de carbone et l'eau supérieur à 1, comprenant les étapes consistant à:

a) mettre en contact la solution aqueuse avec le dioxyde de carbone liquide à une température inférieure à 31,1°C et à une pression relative comprise entre 25,53 et 73,22 bars pour former une phase d'extraction contenant le produit chimique organique, du dioxyde de carbone et un peu d'eau et une phase résiduelle contenant l'eau;

b) séparer la phase d'extraction de la phase résiduelle;

c) évaporer une partie du dioxyde de carbone à partir de la phase d'extraction; et

d) cristalliser et séparer l'hydrate de dioxyde de carbone à partir de la phase d'extraction.